# EUROPEAN PATENT APPLICATION

(11) **EP 2 196 133 A1**
(43) Date of publication of application: **16.06.2010**
(21) Application number: 09252709.2
(22) Date of filing: 01.12.2009
(51) Int. Cl.: A61B 1/005

(54) **Endoscope soft portion and endoscope**

(30) Priority: 10.12.2008 JP 2008314980
(71) Applicant: FUJIFILM Corporation, Minato-ku Tokyo (JP)
(72) Inventor: Fukunaga, Toshiaki, Ashigarakami-gun Kanagawa (JP); Nakamura, Shigeru, Ashigarakami-gun Kanagawa (JP)
(74) Representative: Stevens, Jason Paul

(57) **Abstract**

An endoscope soft portion (30) is formed from: a spiral tube (32) that is formed by spirally winding a strip-shaped member; a mesh tube (34) that is disposed on the outer periphery of the spiral tube (32) and is formed in an annular shape by braiding together fine wires; and an outer sheath member (36) that covers the outer periphery of the mesh tube (34). The mesh tube (34) is formed by a thermoplastic resin material, and a poly-para-xylylene resin layer (35) is coated on the outer peripheral surface of the mesh tube (34). The invention also extends to an endoscope (10) using such an endoscope soft portion.

## Description

The present invention relates to a tubular endoscope soft portion and to an endoscope equipped with this endoscope soft portion.

Medical endoscopes are used to observe organs and the like by inserting an elongate insertion portion inside a body cavity of a patient and to perform various treatments and procedures using accessories inserted inside an accessory insertion channel in the endoscope. For this reason, when an endoscope that has been used once is to be reused in another patient, it is necessary to disinfect and sterilize the endoscope after the end of examination and treatment in order to prevent infection between patients via the endoscope. For disinfection and sterilization, there are methods that use disinfectant solutions, ethylene oxide gas, formalin gas, hydrogen peroxide gas, plasma, ozone, or autoclaving, which is sterilization that uses high-temperature high-pressure steam.

The autoclaving method, which sterilizes endoscopes with high-temperature high-pressure steam, is a sterilization method that has conventionally become widespread. This method has many advantages, such as the reliability of the sterilization effect is high, there is no residual toxicity, and running costs are inexpensive; however, as typical conditions when sterilizing endoscopes with high-temperature high-pressure steam, it is prescribed in ANSI/AAMI ST 37-1992 approved by the American National Standards Institute (ANSI) and published by the Association for the Advancement of Medical Instrumentation (AAMI) that the sterilization process should be at 132° C for 4 minutes in the pre-vacuum type and that the sterilization process should be at 132° C for 10 minutes in the gravity type, and under such an environment, damage to medical equipment can become extremely large.

The soft portion (flexible tube) that configures the insertion portion of an endoscope is, for example, equipped with a spiral tube that is formed in a constant diameter by spirally winding a strip-shaped member, a mesh tube that is formed by braiding together fine wires on the outer periphery of this spiral tube, and an outer sheath layer that covers the outer periphery of this mesh tube. When the mesh tube is formed by braiding together stainless steel wires and the outer sheath layer is formed by a thermoplastic resin material (a thermoplastic elastomer), the mesh tube and the outer sheath member are formed by different materials, so adhesion and bonding between the mesh tube and the outer sheath layer are inferior. When a principal disinfection and sterilization treatment such as autoclaving is repeatedly performed in a state using a thermoplastic elastomer outer sheath layer and a stainless steel wire mesh tube, oxidation and rust occur in the fine wires such as the stainless steel wires and those fine wires deteriorate. When the mesh tube is formed by braiding together stainless steel wires, fraying arises in the mesh tube because of prolonged use under a hostile environment, and a situation arises where the wires are ablated by attack resulting from resistance to humidity and heat, resistance to chemicals and resistance to autoclaving.

In order to prevent this, in Japanese Patent Application Laid-Open Publication (JP-A) No. 2006-334287, there is proposed a configuration where the mesh tube and the spiral tube are formed by a thermoplastic resin such as a polyolefin. However, with the resin described in JP-A No. 2006-334287, it is difficult to raise rigidity, and there cannot be realized the effects of strength with respect to expansion/contraction and torsion being increased by the original mesh tube and strength with respect to crushing of the insertion portion of the endoscope being high. Thus, there is the potential for the insertion portion of the endoscope to end up deforming because of repeated bending.

In consideration of the above-described circumstances, it is an object of at least the preferred embodiments of the present invention to obtain an endoscope soft portion and an endoscope that can control deformation of the insertion portion of the endoscope resulting from oxidation and deterioration of the mesh tube at the time of sterilization with high-temperature high-pressure steam.

According to a first aspect of the present invention, there is provided an endoscope soft portion including: a spiral tube that is formed by spirally winding a strip-shaped member; a mesh tube that is disposed on the outer periphery of the spiral tube and is formed in an annular shape by braiding together fine wires; and an outer sheath member that covers the outer periphery of the mesh tube, wherein the mesh tube is formed by a thermoplastic resin material, and a poly-para-xylylene resin layer is coated on the outer peripheral surface of the mesh tube.

The poly-para-xylylene resin layer is coated on the outer peripheral surface of the mesh tube that is formed by the thermoplastic resin material, so there can be realized the effects of the rigidity of the mesh tube rising, strength with respect to expansion/contraction and torsion being increased by the original mesh tube and strength with respect to crushing of the endoscope soft portion (particularly the insertion portion) being high. Further, the poly-para-xylylene resin layer is coated on the outer peripheral surface of the mesh tube, so oxidation and deterioration of the mesh tube at the time of sterilization with high-temperature high-pressure steam are controlled, and deformation of the endoscope soft portion (particularly the insertion portion) can be controlled.

Preferably, the poly-para-xylylene resin layer may be further coated on the outer peripheral surfaces of the spiral tube in a state where the spiral tube and the mesh tube are incorporated together.

With this configuration, oxidation and deterioration of the mesh tube and the spiral tube at the time of sterilization with high-temperature high-pressure steam are controlled, and deformation of the endoscope soft portion (particularly the insertion portion) can be controlled.

In a further preferred form, the thickness of the poly-para-xylylene resin layer is in the range of 5 µm to 80 µm.

By placing the thickness of the poly-para-xylylene resin layer in the range of 5 µm to 80 µm, bending rigidity can be ensured while maintaining the flexibility of the endoscope soft portion. In contrast, when the thickness of the poly-para-xylylene resin layer is smaller than 5 µm, it becomes difficult to ensure the desired bending rigidity. Further, when the thickness of the poly-para-xylylene resin layer is larger than 80 µm, it becomes difficult to maintain the flexibility of the endoscope soft portion.

The invention also extends to an endoscope whose elongate insertion portion which is inserted inside a body cavity of a patient is formed from an endoscope soft portion as described above.

Thus, the elongate insertion portion which is inserted inside a body cavity of a patient is formed by the endoscope soft portion, so strength with respect to crushing of the endoscope soft portion (particularly the insertion portion) can be raised. Further, the poly-para-xylylene resin layer is coated on the outer peripheral surface of the mesh tube etc., so oxidation and deterioration of the mesh tube etc. at the time of sterilization with high-temperature high-pressure steam are controlled, and deformation of the endoscope soft portion (particularly the insertion portion) can be controlled.

In at least the preferred embodiments of the present invention, strength with respect to crushing of the endoscope soft portion (particular the insertion portion) can be raised. Further, oxidation and deterioration of the mesh tube and the spiral tube at the time of sterilization with high-temperature high-pressure steam are controlled, and deformation of the endoscope soft portion (particularly the insertion portion) can be controlled.

Preferred embodiments of the present invention will now be described in detail based on the following figures and by way of example only, wherein:
FIG. 1 is a general configuration view showing an endoscope that uses an endoscope soft portion in a first embodiment of the present invention;
FIG. 2 is a partially exploded side view showing the configuration of the endoscope soft portion in the first embodiment of the present invention;
FIG. 3 is a partially exploded perspective view showing the configuration of the endoscope soft portion in the first embodiment of the present invention;
FIG. 4 is a cross-sectional view showing the configuration of the endoscope soft portion in the first embodiment of the present invention; and
FIG. 5 is a cross-sectional view showing the configuration of an endoscope soft portion in a second embodiment of the present invention.

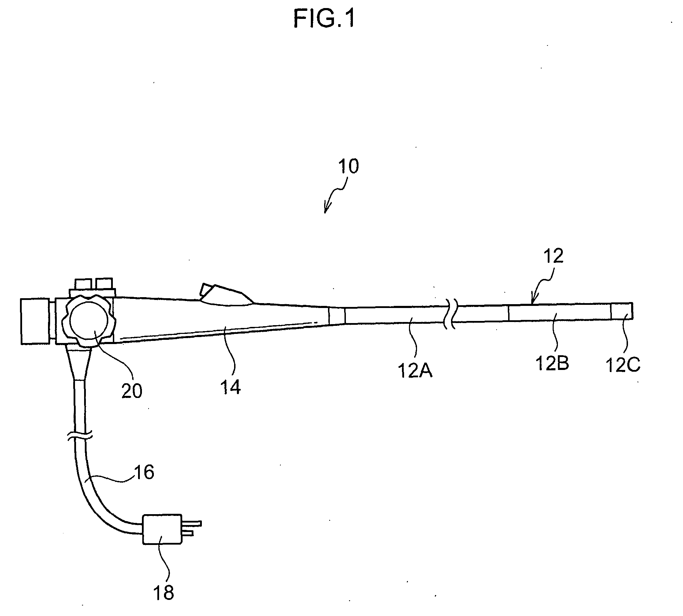

In FIG. 1, there is shown the overall configuration of an endoscope 10 pertaining to a first embodiment of the present invention. As shown in this drawing, the endoscope 10 is equipped with an elongate insertion portion 12 that is inserted inside a body cavity of a patient, and a body operation portion 14 is disposed consecutively with the proximal end portion of the insertion portion 12. An elongate light guide soft portion 16 that is detachably connected to a light source device (not shown) is coupled to the body operation portion 14. A connector portion 18 equipped with a terminal that is connected to the light source device is disposed on the distal end portion of the light guide soft portion 16. Further, an operation knob 20 for operating the insertion portion 12 is disposed on the body operation portion 14.

The insertion portion 12 is equipped with a flexible tube portion 12A serving as an endoscope soft portion that forms the majority of the length portion of the insertion portion 12 in the longitudinal direction (axial direction) from the portion disposed consecutively with the body operation portion 14, an angle portion 12B that is disposed consecutively with the longitudinal direction distal end side of this flexible tube portion 12A, and a distal end portion body 12C that is disposed consecutively with the longitudinal direction distal end side of the angle portion 12B and has a built-in objective optical system and the like. The angle portion 12B is configured such that it can be remotely bent by rotating the operation knob 20 disposed on the body operation portion 14. Further, the light guide soft portion 16 also has substantially the same structure as that of the flexible tube portion 12A of the insertion portion 12.

The flexible tube portion 12A has a length such that the distal end portion body 12C can reach the inside of a predetermined observation target area, and the flexible tube portion 12A is set to a length where it can be kept away from the patient or the like to an extent that it does not disrupt an operator gripping and operating the body operation portion 14. It is necessary for the flexible tube portion 12A to be given flexibility across substantially its entire length, and the flexible tube portion 12A has a structure where particularly the part that is inserted inside a body cavity of a patient has more flexibility. Further, particularly at the portion where the flexible tube portion 12A is disposed consecutively with the body operation portion 14, some rigidity becomes necessary with respect to bending in order to obtain a push-in thrust force when inserting the insertion portion 12 inside a body cavity or the like. Further, it is preferred that the portion where particularly the flexible tube portion 12A is disposed consecutively with the angle portion 12B has more flexibility in order to allow it to follow the curved shape to a certain extent when the angle portion 12B curves.

The flexible tube portion 12A houses, inside a tubular portion, a light guide, an image guide (a signal cable in the case of an electronic endoscope), an accessory insertion channel and air/water tubes that are not shown.

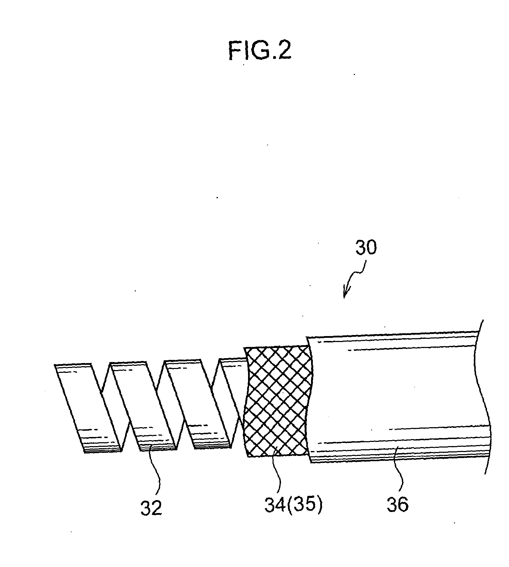

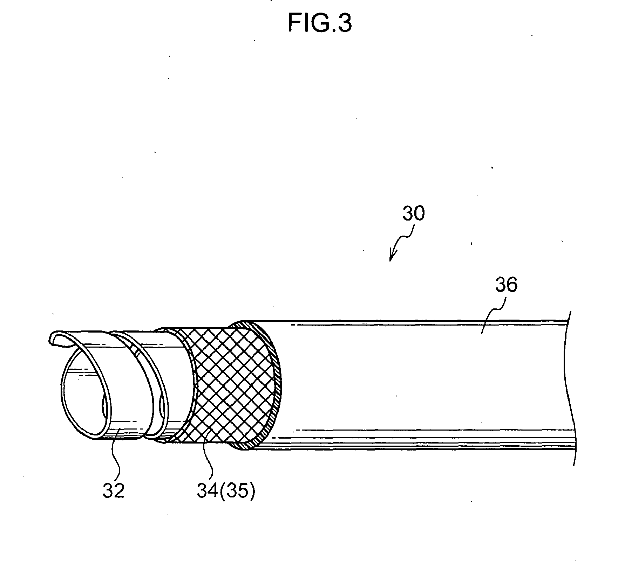

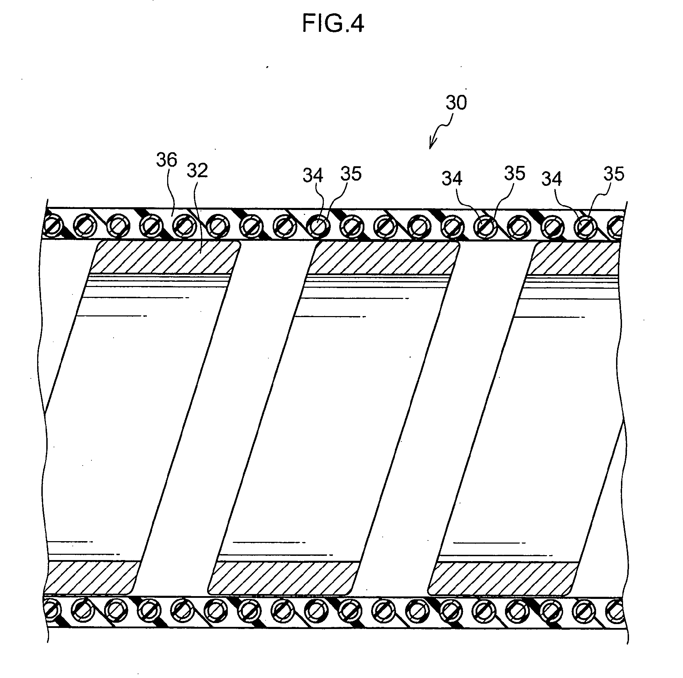

In FIG. 2, an endoscope soft portion 30 that forms the tubular portion of the flexible tube portion 12A is shown in a partial cutaway side view. Further, in FIG. 3, the endoscope soft portion 30 is shown in a partial cutaway perspective view, and in FIG. 4, the endoscope soft portion 30 is shown in a cross-sectional view. As shown in these drawings, the endoscope soft portion 30 is equipped with a spiral tube 32 that is formed by spirally winding a strip-shaped member made of metal, a mesh tube 34 that is formed in an annular shape by braiding together fine wires on the outer periphery of this spiral tube 32, and an outer sheath member 36 that is formed on the outer periphery of this mesh tube 34. The mesh tube 34 is formed by a thermoplastic resin material, and a poly-para-xylylene resin layer 35 is coated on the outer peripheral surface of the thermoplastic resin material.

Next, a method of creating the mesh tube 34 will be described.

The mesh tube 34 is formed by a resin net extrusion device. The resin net extrusion device is not shown in the drawings, but it is equipped with a die portion that has at least one pair of dies having nozzle grooves, a drive portion that drives this die portion, and an extrusion portion that supplies molten resin to the nozzle grooves of the die portion via this drive portion. When the pair of dies are moved relatively with respect to each other by a circular die method or a flat die method, net strands are fused together and nodes are formed at every predetermined interval, so a net is easily formed; thus, the mesh tube 34 is easily formed. The mesh tube 34 is formed in a circular cylinder shape (an annular shape) by a plastic net in which fine wires of the thermoplastic resin material, such as an olefin elastomer such as polypropylene resin or ethylene-polypropylene copolymer resin, are braided together.

As the thermoplastic resin material, it is possible to use another elastomer instead of an olefin elastomer such as polypropylene resin or ethylene-polypropylene copolymer resin, such as, for example, a polyamide elastomer, a fluorinated elastomer, a styrene elastomer, a polyester elastomer, or a polyurethane elastomer.

After this, the mesh tube 34 is hung inside and a coating is applied to the entire outer periphery of the mesh tube 34. That is, poly-para-xylylene resin is applied by chemical vapour deposition. Examples of poly-para-xylylene resin include poly-monochloro-para-xylylene, poly-para-xylylene, poly-dichloro-para-xylylene and fluorinated poly-para-xylylene, and one whose properties are suitable is selected or combined to make a coating agent.

The coating treatment is performed by placing the insertion portion whose assembly has been completed inside a chamber of a chemical vapour deposition device, depressurizing the chamber, feeding vaporized di-para-xylylene through a heating tube into the inside of the chamber, and then leaving the insertion portion in that atmosphere for a predetermined amount of time. The coating thickness can be adjusted by varying the amount of time the insertion portion spends in the resin atmosphere. Thus, the poly-para-xylylene resin layer 35 is formed on the outer peripheral surface of the mesh tube 34. The thickness of the poly-para-xylylene resin layer 35 is preferably in the range of 5 to 80 µm, more preferably in the range of 10 µm to 60 µm, and even more preferably in the range of 15 µm to 40 µm.

The outer sheath member 36 covers and is formed by extrusion moulding on the outer periphery of the member in which the mesh tube 34 (on which the poly-para-xylylene resin layer 35 has been coated) has been disposed on the outer periphery of the spiral tube 32. The endoscope soft portion 30 (flexible tube) is formed by this method.

As described above, the endoscope soft portion 30 is configured by the spiral tube 32, the mesh tube 34 that is disposed on the outer periphery of this spiral tube 32, and the flexible outer sheath member 36 that is disposed on the outer periphery of this mesh tube 34. In other words, this endoscope soft portion 30 is, in order to ensure strength with respect to crushing, formed such that the mesh tube 34 that ensures strength with respect to expansion/contraction and torsion covers the outer periphery of the spiral tube 32 and such that the outer sheath member 36 covers the outer periphery of this mesh tube 34.

The outer sheath member 36 is thinly formed by a material having excellent resistance to chemicals and excellent slidability with respect to a body wall of a patient. The outer sheath member 36 is formed by an olefin elastomer (a thermoplastic resin) such as polypropylene resin or ethylene-polypropylene copolymer resin.

Before forming the outer sheath member 36, it is preferable to make the surface of the poly-para-xylylene hydrophilic to strengthen bonding with the outer sheath member 36 by an atmospheric-pressure plasma treatment such as under a mixed atmosphere of a noble gas and oxygen.

Moreover, it is desirable to apply a silane coupling agent coating layer to at least part of the area between the mesh tube 34 on which the poly-para-xylylene resin layer 35 has been coated and the outer sheath member 36. This is because, when a heat treatment is performed after covering the mesh tube 34 with the outer sheath member 36, a functional group produced by hydrolysis of the silane coupling agent strongly bonds together with the mesh tube 34 and an organic functional group of the silane coupling agent chemically reacts with and strongly joins to the resin mixture that forms the outer sheath member 36. This joint between the mesh tube 34 and the outer sheath member 36 has high resistance with respect to water, so the joint is not damaged even when subjected to autoclave sterilization.

The silane coupling agent can include at least one type of organic functional group selecting from the group comprising a vinyl group, a methacryl group, an epoxy group, an amino group and a mercapto group. Preferably, a silane coupling agent including a methacryl group is used. As described above, these organic functional groups chemically react with the resin mixture because of the heat treatment, whereby the mesh tube 34 and the outer sheath member 36 are strongly joined together.

It will be noted that, after the mesh tube 34 is covered with the outer sheath member 36, it is desirable to administer a heat treatment to the endoscope soft portion 30 (flexible tube) at a temperature equal to or greater than the crystalline melting temperature of the resin mixture. Because of this heat treatment, a graft copolymer within the resin mixture and the mesh tube 34 become strongly bonded. Further, when a silane coupling agent is used as described above, the mesh tube 34 and the outer sheath member 36 are strongly joined together, and it is possible to dramatically improve bonding between the outer sheath member 36 and the coat layer thereon.

Next, the action and effects of the endoscope soft portion 30 will be described.

In the endoscope soft portion 30, the poly-para-xylylene resin layer 35 is coated on the outer peripheral surface of the mesh tube 34 that is formed by the thermoplastic resin material, so there can be realized the effects of the rigidity of the mesh tube 34 being increased, strength with respect to expansion/contraction and torsion being increased by the original mesh tube 34, and strength with respect to crushing of the endoscope soft portion 30 (particularly the insertion portion) being high. Further, the poly-para-xylylene resin layer 35 is coated on the outer peripheral surface of the mesh tube 34, so oxidation and deterioration of the mesh tube 34 at the time of sterilization with high-temperature high-pressure steam are controlled, and deformation of the endoscope soft portion 30 (particularly the insertion portion) can be controlled.

Further, by placing the thickness of the poly-para-xylylene resin layer 35 in the range of 5 µm to 80 µm, bending rigidity can be ensured while maintaining the flexibility of the endoscope soft portion 30. In contrast, when the thickness of the poly-para-xylylene resin layer 35 is smaller than 5 µm, it becomes difficult to ensure the desired bending rigidity. Further, when the thickness of the poly-para-xylylene resin layer 35 is larger than 80 µm, it becomes difficult to maintain the flexibility of the endoscope soft portion. Consequently, it is preferable to place the thickness of the poly-para-xylylene resin layer 35 in the range of 5 µm to 80 µm.

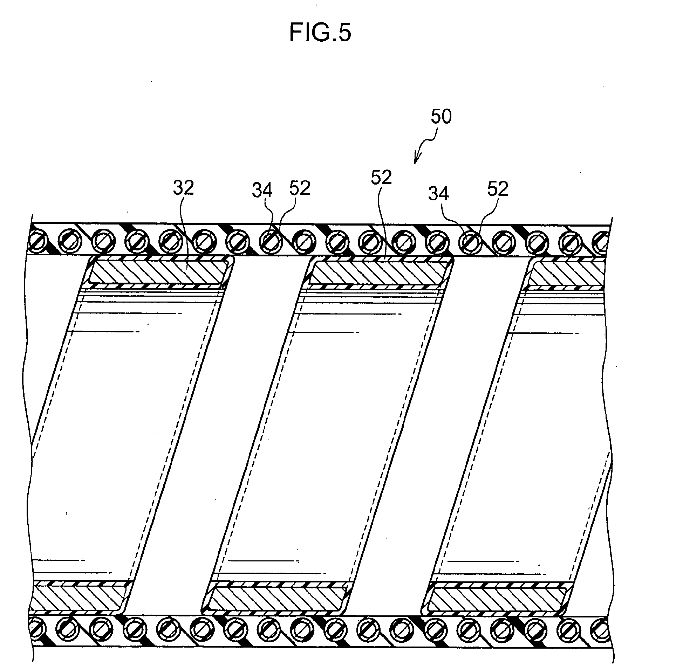

In FIG. 5, there is shown an endoscope soft portion 50 that is used in an endoscope pertaining to a second embodiment of the present invention. It will be noted that the same reference numerals will be given to members that are the same as those of the first embodiment and that redundant description of those same members will be omitted.

As shown in FIG. 5, the endoscope soft portion 50 is equipped with the spiral tube 32 and the mesh tube 34 that is formed by the thermoplastic resin material, and a poly-para-xylylene resin layer 52 is coated on the outer peripheral surfaces of the spiral tube 32 and the mesh tube 34 in a state where the spiral tube 32 and the mesh tube 34 have been incorporated together. The poly-para-xylylene resin layer 52 uses the same material as the poly-para-xylylene resin layer 35 of the first embodiment and is set to substantially the same thickness as that of the poly-para-xylylene resin layer 35.

In the endoscope soft portion 50, the poly-para-xylylene resin layer 52 is coated on the outer peripheral surface of the mesh tube 34 that is formed by a thermoplastic resin material, so there are realized the effects of the rigidity of the mesh tube 34 rising, strength with respect to expansion/contraction and torsion being increased by the original mesh tube 34, and strength with respect to crushing of the endoscope soft portion 50 (particularly the insertion portion) being high. Further, the poly-para-xylylene resin layer 52 is coated on the outer peripheral surfaces of the spiral tube 32 and the mesh tube 34 in a state where the spiral tube 32 and the mesh tube 34 have been incorporated together, so oxidation and deterioration of the mesh tube 34 and the spiral tube 32 at the time of sterilization with high-temperature high-pressure steam are controlled, and deformation of the endoscope soft portion (particularly the insertion portion) can be controlled.

It will be noted that the shapes and arrangement of the spiral tube 32 and the mesh tube 34 in the endoscope soft portion are not limited to the first and second embodiments and may also have other configurations.

## Claims

1. An endoscope soft portion (30) comprising:
a spiral tube (32) that is formed by spirally winding a strip-shaped member;
a mesh tube (34) that is disposed on the outer periphery of the spiral tube (32) and is formed in an annular shape by braiding together fine wires; and
an outer sheath member (36) that covers the outer periphery of the mesh tube (34),
wherein the mesh tube (34) is formed by a thermoplastic resin material, and a poly-para-xylylene resin layer (35) is coated on the outer peripheral surface of the mesh tube (34).

2. The endoscope soft portion according to claim 1,
wherein the poly-para-xylylene resin layer (52) is further coated on the outer peripheral surfaces of the spiral tube (32) in a state where the spiral tube (32) and the mesh tube (34) are incorporated together.

3. The endoscope soft portion according to claim 1 or claim 2,
wherein the thickness of the poly-para-xylylene resin layer (35, 52) is in the range of 5 µm to 80 µm.

4. An endoscope (10) whose elongate insertion portion (12A) which is inserted inside a body cavity of a patient is formed from an endoscope soft portion (30) according to any one of claims 1 to 3.
